(19) Europäisches Patentamt
European Patent Office
Office européen des brevets

(11) Veröffentlichungsnummer: **0 310 689 A1**

# (12) EUROPÄISCHE PATENTANMELDUNG

(21) Anmeldenummer: **87114496.0**

(51) Int. Cl.4: **C07D 487/08**

(22) Anmeldetag: **05.10.87**

(43) Veröffentlichungstag der Anmeldung: **12.04.89 Patentblatt 89/15**

(84) Benannte Vertragsstaaten: **BE CH DE FR GB LI NL**

(71) Anmelder: **BASF Aktiengesellschaft**
**Carl-Bosch-Strasse 38**
**D-6700 Ludwigshafen(DE)**

(72) Erfinder: **Hoelderich, Wolfgang, Dr.**
**18c Mannheimer Strasse**
**D-6710 Frankenthal(DE)**
Erfinder: **Schneider, Kurt, Dr.**
**Auf dem Koeppel**
**D-6702 Bad Dürkheim(DE)**
Erfinder: **Best, Walter, Dr.**
**Im Spiess 2**
**D-6714 Weisenheim(DE)**

## (54) Verfahren zur Herstellung von 1,4-Diazabicyclo(2,2,2)-octanen.

(57) Die Anmeldung betrifft ein Verfahren zur Herstellung von 1,4-Diazabicyclo(2,2,2)-octan und C-substituierten 1,4-Diazabicyclo(2,2,2)-octanen der Formel (I)

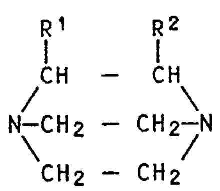

(I),

in der $R^1$ und $R^2$ Wasserstoff-, Alkyl- mit 1 bis 4 C-Atomen oder Alkenylreste mit 1 bis 4 C-Atomen bedeuten können, aus heterocyclischen Amine der Formel (II)

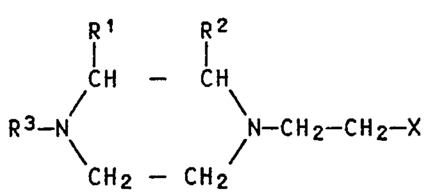

(II),

in der $R^1$ und $R^2$ obige Bedeutung haben und $R^3$ Wasserstoff- oder Hydroxyethyl- oder Aminoethyl- und X Hydroxyl-, Amino-, Hydroxyethyl- oder Aminoethylreste bedeuten, in Gegenwart von Boro- und/oder Eisensilikatzeolithen als Katalysatoren.

Besonders geeignet als Katalysatoren sind die Boro- und Eisensilikatzeolithe des Pentasiltyps.

EP 0 310 689 A1

## Verfahren zur Herstellung von 1,4-Diazabicyclo(2,2,2)-octanen

Die vorliegende Erfindung betrifft ein Verfahren zur Herstellung von 1,4-Diazabicyclo(2,2,2)-octan (DABCO) und dessen C-substituierte Derivate durch Umsetzung heterocyclischer Amine in Gegenwart von Boro- und/oder Eisensilikatzeolithen als Katalysatoren.

Es ist bekannt, daß man DABCO und seine Derivate aus heterocyclischen Aminen heterogenkatalysiert herstellen kann. Als Katalysatoren hat man bisher die Phosphate des Ca, Sr, Ba, Zn, La, Al, Co, Ni, Ce verwendet, jedoch unbefriedigende Ausbeuten erzielt.

Es ist ferner bekannt, Aluminiosilikatzeolithe vom ZSM-Typ als Heterogenkatalysatoren für diese Reaktion einzusetzen (EP 158 319). Die Ausbeuten sind klein; bei niedrigem Umsatz werden zwar hohe Selektivitäten, bei hohem Umsatz jedoch niedrige Selektivitäten gefunden. Weiterhin weist das Reaktionsprodukt eine Reihe unerwünschter Nebenprodukte wie N-Alkylpiperazine und Pyrazin-Derivate auf. Die Nebenprodukte haben physikalische und chemische Eigenschaften ähnlich dem DABCO und lassen sich daher mit konventionellen physikalischen Methoden wie Destillation und Kristallisation nur schwer abtrennen. Für die Weiterverarbeitung werden an die Reinheit des DABCO hohe Anforderungen gestellt und erfordern daher eine aufwendige Reinigung.

Es war daher die Aufgabe gestellt, Katalysatoren zu finden, die hohe Ausbeuten (Umsatz x Selektivität) an den gewünschten 1,4-Diazabicyclo(2,2,2)-octanen liefern und die oben genannten Nachteile vermeiden.

Es wurde gefunden, daß man 1,4-Diazabicyclo(2,2,2)-octan und C-substituierte 1,4-Diazabicyclo(2,2,2)-octane der Formel (I)

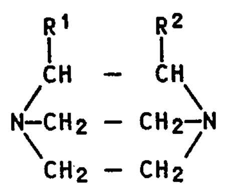

(I),

in der $R^1$ und $R^2$ Wasserstoff-, Alkyl- mit 1 bis 4 C-Atomen oder Alkenylreste mit 1 bis 4 C-Atomen bedeuten, in gewünschter Weise erhält, wenn man heterocyclische Amine der Formel (II)

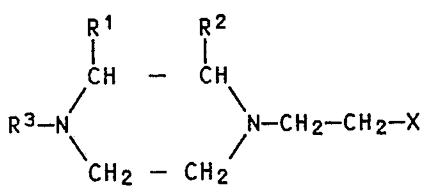

(II),

in der $R^1$ und $R^2$ obige Bedeutung haben und $R^3$ Wasserstoff- oder Hydroxyethyl- oder Aminoethyl- und X Hydroxyl-, Amino-, Hydroxyethyl- oder Aminoethylreste bedeuten, in Gegenwart von Boro- und/oder Eisensilikatzeolithen als Katalysatoren umsetzt.

Vorzugsweise können erfindungsgemäß Verbindungen wie z.B. Monohydroxyethylpiperazin, Dihydroxyethylpiperazin, Monoaminoethylpiperazin, Aminoethylhydroxyethylpiperazin umgesetzt werden.

Als Katalysatoren für das erfindungsgemäße Verfahren verwendet man die Zeolithe zweckmäßig in der aciden Form. Zeolithe sind kristalline Aluminiumsilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si-und Al-Atome zu Sauerstoff beträgt 1 : 2. Die Elektrovalenz der Aluminium enthaltenden Tetraeder ist durch Einschluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithode oder deren Gemische sowie Alumino-, Boro, Gallium- und Eisengermanatzeolithe oder deren Gemische.

Insbesondere eignen sich die Boro- und Eisensilikatzeolithe des Pentasiltyps für das erfindungsgemäße Verfahren.

Borosilikatzeolithe kann man z.B. bei 90 bis 200 °C unter autogenem Druck synthetisieren, indem man eine Borverbindung, z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Hierzu gehören auch die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid in wäßriger Aminlösung, insbesondere 1,6-Hexandiamin, mit oder ohne Alkali- oder Erdalkalizusatz bei 100 bis 200 °C unter autogenem Druck.

Die so hergestellten Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90 : 10 bis 40 : 60 Gew.-% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25 : 75 bis 90 : 5, bevorzugt 75 : 25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Eisen- bzw., Borosilikatzeolith direkt nach der Trocknung verformt wird und erst nach der Verformung einer Calcinierung unterworfen wird. Die hergestellten Eisen- und Borosilikatzeolithe können in reiner Form, ohne Binder, als Stränge oder Tabletten eingesetzt werden, wobei als Verstrangungs- oder Peptisierungshilfsmittel, z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Liegt der Zeolith aufgrund der Art seiner Herstellung nicht in der katalytisch aktiven, aciden H-Form vor, sondern z.B. in der Na-Form, dann kann diese durch Ionenaustausch, z.B. mit Ammoniumionen und anschließende Calcinierung oder durch Behandlung mit Säuren vollkommen oder partiell in die gewünschte H-Form überführt werden.

Wenn bei der erfindungsgemäßen Verwendung der zeolithischen Katalysatoren eventuell eine durch Koksabscheidung bedingte Desaktivierung eintritt, empfiehlt es sich, die Zeolithe durch Abbrennen der Koksablagerung mit Luft oder mit einem Luft/$N_2$-Gemisch bei 400 bis 550 °C, bevorzugt 500 °C, zu regenerieren. Die Zeolithe erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Um eine möglichst hohe Selektivität, hohe Umsätze sowie lange Standzeiten zu erreichen, ist es vorteilhaft, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle werden Alkalimetalle wie Li, Cs, K, Erdalkalimetall wie Mg, Ca, Sr, Metalle der 3., 4. und 5. Hauptgruppe wie Al, Ga, Ge, Sn, Pb, Bi, Übergangsmetalle der 4. - 8. Nebengruppe wie Ti, Zr, V, Nb, Cr, Mo, W, Mn, Re, Fe, Ru, Os, Co, Rh, Sr, Ni, Pd, Pt, Übergangsmetalle der 1. und 2. Nebengruppe wie Cu, Ag, Zn, Seltene Erdmetalle wie La, Ce, Pr, Nd, Er, Yb und U eingesetzt.

Zweckmäßigerweise führt man die Dotierung so durch, daß man den verformten Zeolithen in einem Steigrohr vorlegt und bei 20 bis 100 °C eine wäßrige oder ammonialkalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Eine mögliche Ausführungsform besteht z.B. darin, daß man $Cu(NO_3)_2 \times H_2O$ oder $Ni(NO_3)_2 \times 6\ H_2O$ oder $Ce(NO_3)_3 \times 6\ H_2O$ oder $La(NO_3)_2 \times 6\ H_2O$ oder $Cs_2CO_3$ in Wasser löst und mit dieser Lösung den verformten oder unverformten Zeolith eine gewisse Zeit, z.B. 30 Minuten, tränkt. Die eventuell überstehende Lösung wird im Rotationsverdampfer von Wasser befreit. Danach wird der getränkte Zeolith bei etwa 150 °C getrocknet und bei etwa 550 °C calciniert. Dieser Tränkvorgang kann mehrmals hintereinander vorgenommen werden, um den gewünschten Metallgehalt einzustellen.

Es ist auch möglich, ein wäßrige $Ni(NO_3)_2$-Lösung oder ammoniakalische $Pd(NO_3)_2$-Lösung herzustellen und darin den reinen pulverförmigen Zeolithen bei 40 bis 100 °C unter Rühren etwa 24 h aufzuschlämmen. Nach Abfiltrieren, Trocknen bei etwa 150° C und Calcinierung bei etwa 500° C kann das so gewonnene zeolithische Material mit oder ohne Bindemittel zu Strängen, Pellets oder Wirbelgut weiterverarbeitet werden.

Ein Ionenaustausch des in der H-Form vorliegenden Zeolithen kann so vorgenommen werden, daß man den Zeolithen in Strängen oder Pellets in einer Kolonne vorlegt und darüber z.B. eine wäßrige $Ni(NO_3)_2$-Lösung oder ammoniakalische $Pd(NO_3)_2$-Lösung bei leicht erhöhter Temperatur zwischen 30 und 80° C im Kreislauf 15 bis 20 h leitet. Danach wird mit Wasser ausgewaschen, bei etwa 150° C getrocknet und bei etwa 550° C calciniert. Bei manchen metalldotierten Zeolithen, z.B. Pd-, Cu-, Ni-dotierten Zeolithen ist eine Nachbehandlung mit Wasserstoff vorteilhaft.

Eine weitere Möglichkeit der Modifizierung besteht darin, daß man das zeolithische Material - verformt oder unverformt - einer Behandlung mit Säuren wie Salzsäure, Flußsäure und Phosphorsäure und/oder Wasserdampf unterwirft. Dabei geht man vorteilhaft, z.B. so vor, daß man Zeolithe in Pulverform mit 1 n Phosphorsäure 1 Stunde bei 80° C behandelt. Nach der Behandlung wird mit Wasser gewaschen, bei 110° C/16 Stunden getrocknet und bei 500° C/20 Stunden calciniert. Nach einer anderen Arbeitsweise behandelt man Zeolithe vor oder nach ihrer Verformung mit Bindemittel, z.B. 1 bis 3 Stunden bei Temperaturen von 60 bis 80° C mit einer 3 bis 25 Gew.-%igen, insbesondere 12 bis 20 Gew.-%igen wäßrigen Salzsäure. Anschließend wird der so behandelte Zeolith mit Wasser gewaschen, getrocknet und bei 400° C bis 500° C calciniert.

Eine besondere Ausführungsform für die Säurebehandlung besteht darin, daß man das zeolithische Material vor seiner Vorformung bei erhöhter Temperatur mit Flußsäure, die im allgemeinen als 0,001 n bis 2 n, vorzugsweise 0,05 n bis 0,5 n Flußsäure eingesetzt wird, behandelt, beispielsweise durch Erhitzen unter Rückfluß über einen Zeitraum von im allgemeinen 0,5 bis 5, vorzugsweise 1 bis 3 Stunden. Nach Isolierung, z.B. durch Abfiltrieren und Auswaschen des zeolithischen Materials, wird dieses zweckmäßig, z.B. bei Temperaturen von 100 bis 160° C, getrocknet und bei Temperaturen von im allgemeinen 450° C bis 600° C calciniert. Gemäß einer weiteren bevorzugten Ausführungsform für die Säurebehandlung, wird das zeolithische Material nach einer Verformung mit Bindemittel bei erhöhter Temperatur, zweckmäßig bei Temperaturen von 50 bis 90° C, vorzugsweise 60 bis 80° C, über einen zeitraum von 0,5 bis 5, vorzugsweise mit 12 bis 20 Gew.-%iger Salzsäure, behandelt. Zweckmäßig wird das zeolithische Material anschließend ausgewaschen, bei Temperaturen von 100 bis 160° C getrocknet und bei Temperaturen von 450 bis 600° C calciniert. Einer HF-Behandlung kann sich auch eine HCl-Behandlung anschließen.

Nach einer anderen Arbeitsweise kann man Zeolithe durch Aufbringen von Phosphorverbindungen, wie Trimethoxiphosphat, Trimethoxiphosphin, primärem, sekundärem oder tertiärem Natriumphosphat modifizieren. Besonders vorteilhaft hat sich die Behandlung mit primärem Natriumphosphat erwiesen. Hierbei werden die Zeolithe in Strang-, Tabletten- oder Wirbelgut-Form mit wäßriger $NaH_2PO_4$-Lösung getränkt, bei 110° C getrocknet und bei 500° C calciniert.

Die Katalysatoren können wahlweise als 2 bis 4 mm Stränge oder als Tabletten mit 3 bis 5 mm Durchmesser oder als Pulver mit Teilchengrößen von 0,1 bis 0,5 mm oder als Wirbelkontakt eingesetzt werden.

Die Umwandlung wird bevorzugt in der Gasphase bei 200 bis 550° C, insbesondere 300 bis 450° C, und einer Belastung WHSV = 0,1 bis 20 $h^{-1}$, insbesondere 0,5 bis 5 $h^{-1}$ (g Ausgangsstoff/g Katalysator und Stunde). Die Reaktion wird im Festbett oder Wirbelbett durchgeführt. Im allgemeinen steigt der Umsatz mit steigender Temperatur stark an, während die Selektivität in einem bestimmten Temperaturbereich nur wenig zurückgeht. Die Reaktion kann auch in der Flüssigphase (Suspension-, Riesel- oder Sumpffahrweise) durchgeführt werden. Das Verfahren sind in der Regel bei Normaldruck oder erhöhtem Druck und vorzugsweise kontinuierlich, aber auch diskontinuierlich durchgeführt.

Schwerflüchtige oder feste Ausgangsstoffe werden in gelöster Form, z.B. in Wasser-, THF-, Toluol- oder Petrolether-Lösung eingesetzt. Allgemein ist auch eine Verdünnung mit Lösungsmitteln oder Inertgasen wie $N_2$, Ar, möglich.

Nach der Umsetzung werden die entstandenen 1,4-Diazabicyclo(2,2,2)-octane nach üblichen Verfahren, z.B. durch Destillation aus dem Reaktionsgemisch isoliert; nichtumgesetzte Ausgangsstoffe werden gegebenenfalls in die Umsetzung zurückgeführt.

Beispiele 1 bis 8

Die Reaktion wird unter isothermen Bedingungen in einem Rohrreaktor (Wendel 0,6 cm, 90 cm Länge) in der Gasphase mindestens 6 Stunden lang durchgeführt. Die Trennung und Charakterisierung der Reaktionsprodukte erfolgt nach üblichen Methoden. Die quantitative Bestimmung der Reaktionsprodukte und der Ausgangsstoffe erfolgt gaschromatographisch.

Die in den Beispielen verwendeten Katalysatoren sind:

Katalysator A

Ein Borosilikatzeolith des Pentasil-Typs wird in einer hydrothermalen Synthese aus 640 g hochdispersem $SiO_2$, 122 g $H_3BO_3$, 8 kg einer wäßrigen 1,6-Hexandiamin-Lösung (Mischung 50 : 50 Gew.%) bei 170 °C unter autogenem Druck in einem Rührautoklaven hergestellt. Nach Abfiltrieren und Auswaschen wird das kristalline Reaktionsprodukt bei 100 °C/24 h getrocknet und bei 500 °C/24 h calciniert. Dieser Borosilikatzeolith setzt sich zusammen aus 94,2 Gew.% $SiO_2$ und 2,3 Gew.% $B_2O_3$.

Mit diesem Material werden durch Verformen mit Verformungshilfsmitteln 2 mm-Stränge hergestellt, die bei 110 °C/16 h getrocknet und bei 500 °C/24 h calciniert werden.

Katalysator B

Ein Eisensilikatzeolith des Pentasiltyps wird unter hydrothermalen Bedingungen bei autogenem Druck und 165 °C aus 273 g Wasserglas, gelöst in 253 g einer wäßrigen 1,6-Hexandiamin-Lösung (Mischung 50 : 50 Gew.%) und 31 g Eisensulfat, gelöst in 21 g 96 %iger Schwefelsäure und 425 g Wasser in einem Rührautoklaven während 4 Tagen synthetisiert. Der Zeolith wird abfiltriert, ausgewaschen, bei 110 °C/24 h getrocknet und bei 500 °C/24 h calciniert. Man erhält einen Eisensilikatzeolith mit einem $SiO_2/Fe_2O_3$-Verhältnis von 17,7 und einem $Na_2O$-Gehalt von 1,2 Gew.%. Der Katalysator wird mit hochdispersem $SiO_2$ im Gewichtsverhältnis 80 : 20 zu 2,5 mm-Strängen verstrangt, bei 110 °C/16 h getrocknet und bei 500 °C/24 h calciniert.

Katalysator C

Katalysator C wird durch Imprägnieren von Katalysator A mit einer $Pd(NO_3)_2$-/$Ce(NO_3)_3$-Lösung erhalten. Nach Trocknung bei 130 °C/2 h und Calcination bei 540 °C/2 h beträgt der Pd-Gehalt 1,3 Gew.% und der Ce-Gehalt 3,6 Gew.%.

Katalysator D

Katalysator D erhält man, indem man den Borosilikatzeolithen aus Katalysator A mit hochdispersem $SiO_2$ im Gewichtsverhältnis 70 : 30 zu 2 mm-Strängen verformt, bei 110 °C/16 h trocknet und bei 500 °C/16 h calciniert. Diese Stränge werden mit einer wäßrigen $NaH_2PO_4$-Lösung imprägniert, bei 110 °C getrocknet und bei 500 °C/14 h calciniert. Der Na-Gehalt beträgt 5 Gew.% und der P-Gehalt 7,5 Gew.%.

Katalysator E (Vergleichskatalysator)

Ein Aluminosilikathzeolith ZSM 5 wird nach US-PS 3 702 886, Beispiel 1, synthetisiert. Dieser ZSM 5-Zeolith wird mit Boehmit im Gewichtsverhältnis 60 : 40 verformt, bei 110 °C getrocknet und bei 500 °C/16 h calciniert. Die Stränge werden mit 20 %iger $NH_4Cl$-Lösung bei 80 °C/2 h nach üblicher Methode ionenausgetauscht bis der Na-Gehalt 0,02 Gew.% (nach Trocknung bei 110 °C und Calcination bei 500 °C/5 h) beträgt.

In der nachfolgenden Tabelle 1 sind die mit den oben beschriebenen Katalysatoren erzielten Versuchsergebnisse aufgeführt.

Einsatzlösung I: Monohydroxyethylpiperazin in Wasser gelöst 25 g : 75 g.

Einsatzlösung II: Gemisch aus Mono- und Dihydroxyethylpiperazin (Gewichtsverhältnis 20 : 80) in Wasser gelöst 25 g : 75 g.

Tabelle 1

| Beispiel | 1 | 2 | 3 | 4 | 5* | 6 |
|---|---|---|---|---|---|---|
| Einsatzlösung | I | I | I | I | I | II |
| Katalysator | A | B | C | D | E | A |
| Temperatur | 400 °C | 400 °C | 400 °C | 400 °C | 400 °C | 400 °C |
| WHSV | 1,5 $h^{-1}$ | 1,5 $h^{-1}$ | 1,5 $h^{-1}$ | 3 $h^{-1}$ | 2 $h^{-1}$ | 1,5 $h^{-1}$ |
| Umsatz % | 98,9 | 99,7 | 99,7 | 99,9 | 99,6 | 99,8 |
| Selektivität % | | | | | | |
| DABCO | 65,6 | 73,5 | 76,8 | 83,2 | 68,5 | 68,3 |
| Piperazin | 7,3 | 10,3 | 5,9 | 9,9 | 12,4 | 9,4 |

* Vergleichsbeispiel

Tabelle 1 - Forts.

| Beispiel | 7 | 8 |
|---|---|---|
| Einsatzlösung | II | II |
| Katalysator | C | D |
| Temperatur | 400 °C | 400 °C |
| WHSV | 2 $h^{-1}$ | 2 $h^{-1}$ |
| Umsatz % | 99,6 | 99,8 |
| Selektivität % | | |
| DABCO | 67,8 | 84,9 |
| Piperazin | 6,5 | 3,9 |
| * Vergleichsbeispiel | | |

## Ansprüche

1. Verfahren zur Herstellung von 1,4-Diazabicyclo(2,2,2)-octan und C-substituierten 1,4-Diazabicyclo-(2,2,2)-octanen der Formel (I)

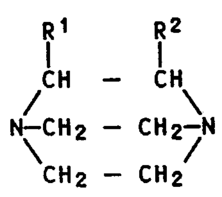

(I),

in der $R^1$ und $R^2$ Wasserstoff-, Alkyl- mit 1 bis 4 C-Atomen oder Alkenylreste mit 1 bis 4 C-Atomen bedeuten können, dadurch gekennzeichnet, daß man man heterocyclische Amine der Formel (II)

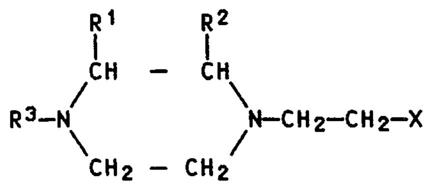

(II),

in der $R^1$ und $R^2$ obige Bedeutung haben und $R^3$ Wasserstoff- oder Hydroxyethyl- oder Aminoethyl- und X Hydroxyl-, Amino-, Hydroxyethyl- oder Aminoethylreste bedeuten, in Gegenwart von Boro- und/oder Eisensilikatzeolithen als Katalysatoren umsetzt.

2. Verfahren nach Anspruch 1, dadurch gekennzeichnet, daß man Monohydroxyethylpiperazin oder Dihydroxyethylpiperazin oder deren Gemische als Ausgangsstoffe umsetzt.

3. Verfahren nach den Ansprüchen 1 und 2, dadurch gekennzeichnet, daß man als Katalysatoren Zeolithe des Pentasiltyps verwendet.

4. Verfahren nach den Ansprüchen 1 bis 3, dadurch gekennzeichnet, daß man als Katalysatoren Borosilikatzeolithe des Pentasiltyps verwendet.

5. Verfahren nach den Ansprüchen 1 bis 4, dadurch gekennzeichnet, daß man mit Seltenen Erden und/oder Übergangsmetallen und/oder Alkali- und/oder Erdalkalimetallen und/oder Phosphorverbindungen modifizierte Boro- und/oder Eisensilikatzeolithe als Katalysatoren verwendet.

Europäisches Patentamt

# EUROPÄISCHER RECHERCHENBERICHT

Nummer der Anmeldung

EP 87 11 4496

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | DE-A-2 434 913 (SHUNAN PETROCHEMICAL CO., LTD.)<br>* Ansprüche 1, 2 * | 1,2 | C 07 D 487/08 |
| D,A | EP-A-0 158 319 (UNION CARBIDE CORP.)<br>* Ansprüche 1, 4 * | 1,2 | |
| A | DD-A- 206 896 (VEB LEUNA-WERKE "WALTER ULBRICHT")<br>* Anprüche 1, 3 * | 1,2 | |
| A | CHEMICAL ABSTRACTS, Band 83, Nr. 21, 24. November 1975, Seite 585, Zusammenfassungsnr. 179111a, Columbus, Ohio, US; & JP - A - 75 58096 | 1,2 | |
| D,A | EP-A-0 034 727 (BASF AG)<br>* Anspruch 1 * | 1 | |
| D,A | EP-A-0 046 504 (BASF AG)<br>* Anspruch 1 * | 1 | |

RECHERCHIERTE SACHGEBIETE (Int. Cl.4)

C 07 D 487/00

Der vorliegende Recherchenbericht wurde für alle Patentansprüche erstellt

| Recherchenort | Abschlußdatum der Recherche | Prüfer |
|---|---|---|
| BERLIN | 11-05-1988 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)